# EUROPÄISCHE PATENTSCHRIFT

(11) **EP 4 011 320 B1**
(45) Veröffentlichungstag und Bekanntmachung des Hinweises auf die Patenterteilung: **20.09.2023**
(21) Anmeldenummer: 20213108.2
(22) Anmeldetag: 10.12.2020
(51) Int. Cl.: A61C 5/90

(54) **FOLIENSPANNELEMENT**
FILM CLAMPING ELEMENT
ÉLÉMENT TENDEUR DE FEUILLE

(43) Veröffentlichungstag der Anmeldung: 15.06.2022
(73) Patentinhaber: Ivoclar Vivadent AG, 9494 Schaan (LI)
(72) Erfinder: Lichtensteiger, Markus, 9462 Montlingen (CH)
(74) Vertreter: Baldus, Oliver

(56) Entgegenhaltungen:
- US-A1- 2017 196 659
- US-A1- 2018 014 914
- US-A1- 2020 188 060

## Beschreibung

Die Erfindung betrifft ein Folienspannelement, gemäß dem Oberbegriff von Anspruch 1. Ein derartiges Folienspannelement wird seit über zehn Jahren unter dem Namen OPTRA-GATE erfolgreich eingesetzt, um den freien Zugang zum Patientenmund bei dentalen Bearbeitungen im Mundraum eines Patienten zu ermöglichen. Es besteht aus einem Vestibulärring und einem Lippenring, zwischen denen sich eine Folie erstreckt. Bei diesem Element ist die Folie gegenüber beiden Ringen verschieblich gelagert und elastisch. Zur Vermeidung von allergischen Beeinträchtigungen ist sie latexfrei ausgebildet.

Ein derartiges Folienspannelement ist dafür bestimmt, zu schützende Bereiche im Mundinnenraum des Patienten abzudecken. Dies schützt zum einen den Zahnarzt vor Infektionen, zum anderen aber den Patienten. Nach Applikation muss ein eingesetztes Folienspannelement entsorgt werden. Bereits daher ist es wichtig, dass es preisgünstig und mit geringem Materialaufwand gefertigt werden kann.

Um den Zugang zum Mund des Patienten zu gewährleisten, bestehen beim bislang bekannten Stand der Technik sowohl der Lippenring als auch der Vestibulärring des Folienspannelements aus einem vergleichsweise steifen Kunsttstoffmaterial.

Hiermit ist es sichergestellt, dass der Mund des Patienten in ausreichendem Maße offen gehalten werden kann. Während die steife Ausgestaltung einen Lippenring in der Regel als nicht besonders unangenehm empfunden wird, wurde bei den Folienspannelementen der ersten Generation patientenseitig darüber geklagt, dass der harte Vestibulärring am Zahnfleisch des Patienten und im gesamten Vestibulärbereich unangenehm ist.

Um zu verhindern, das die Ablehnung durch die Patienten überhand nahm, hat man in der zweiten Generation der Folienspannelemente den Vestibulärring an den relevanten Bereichen aufwendig gepolstert, indem ein weicheres Kunststoffmaterial dort aufgebracht wurde. Die recht teure Lösung wird nach wie vor eingesetzt, und das, obwohl der Materialeinsatz wesentlich größer ist und auch die Fertigung höhere Kosten verursacht.

Ein solches Folienspannelement lässt sich beispielsweise der EP 3 708 112 A1 oder US2017196659 entnehmen.

Aus Kostengründen wird gerne auf die als unangenehm empfundene Version des Folienspannelements mit hartem Vestibulärring zurückgegriffen.

Demgegenüber liegt die Erfindung die Aufgabe zu Grunde, ein Folienspannelement gemäß dem Oberbegriff von Anspruch 1 zu schaffen, das einen hervorragenden Tragekomfort bietet, ohne dass die Herstellungskosten größer wären, wobei es aber dennoch zugleich, trotz der Realisierung als Einwegteil, und damit trotz optimaler Schutzeigenschaften vor Infektionen, die Umwelt weniger belastet wird.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, dass das Folienspannelement zwei Ringe aufweist, nämlich einen Lippenring und einen Vestibulärring. Der Lippenring ist dafür bestimmt, in der Anwendung des Folienspannelements extraoral angeordnet zu sein, und der Vestibulärring ist dafür bestimmt, intraoral angeordnet zu sein, nämlich im Vestibulum des Patienten.

Erfindungsgemäß sind beide Ringe hohl. Sie sind mit einem Gas gefüllt, insbesondere mit Luft, wobei das Gas unter Druck steht. Hierunter ist zu verstehen, das der Innendruck in den Ringen mindestens so groß wie der Umgebungsdruck ist, oder größer.

Beide Ringe sind bevorzugt als Schläuche ausgebildet und bilden je geschlossene Hohlräume. Erfindungsgemäß günstig ist es, dass beide Ringe aus dem gleichen Material wie die Folie sind, die sich zwischen den Ringen erstreckt. Die Folie kann sich auch über den Vestibulärring hinaus erstrecken und dann an dem Vestibulärring umgeschlagen sein.

Die Folie und beide Ringe bestehen aus einem hochelastischem Material, wie es an sich bekannt ist. Bevorzugt sind die Ringe und Folie zueinander einstückig.

Es ist auch möglich, einen Druckluftkanal vorzusehen, der die Hohlräume der Ringe miteinander verbindet. Die Ringe bilden zusammen mit dem Druckluftkanal dann einen einzigen geschlossen Hohlraum.

Einer der Ringe, bevorzugt der Lippenring, kann mit einem Druckluftanschluss versehen sein. Damit ist es möglich, diesen Ring und bevorzugt auch den Vestibulärring unter einen beliebigen gewünschten Druck zu setzen.

Mit zunehmenden Druck wird jeder Ring steifer und mit geringerem Druck wird jeder Ring weicher und leichter zu verformen.

Erfindungsgemäß ist es vorgesehen, dass beide Ringe im Einsatz eine solche Steifheit haben, dass sich die Folie zwischen ihnen aufspannen lässt und das Folienspannelement sich unter leichtem manuellen Zusammendrücken des Vestibulärrings in eine etwas ovale Form in den Mund des Patienten einführen lässt.

In entspannten Zustand weisen beide Ringe, wenn sie unter Druck stehen, bevorzugt im wesentlichen eine Kreisform auf.

Nachdem beide Ringe und die Folie einstückig sind, ist es ohne weiteres möglich, das gesamte Folienspannelement rasch per Spritzguss zu erzeugen. Beliebige andere Herstellungsarten sind ebenso möglich.

Typischerweise weist der Behandlungsplatz eines Zahnarztes eine Druckluftquelle auf. Diese Druckluftquelle lässt sich vom Zahnarzt beispielsweise von Hand oder mit dem Fuß betätigen und kann über einen entsprechenden Adapter auch dazu verwendet werden, den Lippenring und den Vestibulärring aufzublasen. Durch die Wahl der Dauer der Druckluftabgabe lässt sich die Steifheit der Ringe einstellen. Wenn die Druckluftquelle ohnehin hinsichtlich des angegebenen Drucks und/oder des abgegebenen Luftstroms einstellbar ist, lässt sich diese Einstellmöglichkeit auch für das erfindungsgemäße Aufblasen von Lippenring und Vestibulärring verwenden.

Die Ringe können je nach Wahl ihrer Materialstärke eine Form haben, die sich durch das Aufblasen nur wenig ändert, so dass die Ringe durch die Druckluft lediglich prall gefüllt sind. Es ist aber auch möglich, die Ringe oder mindestens einer der Ringe gleichsam wie einen schlauchförmigen Luftballon durch das Aufblasen zu expandieren.

In dieser Ausführungsform lässt sich der Querschnitts-Durchmesser jedes Ringes beispielsweise auf das Dreifache vergrößern.

Die Wirkung der Druckluft auf die Ringe hängt im starken Maße von der Materialstärke der Ringe ab. Wenn beispielsweise die Folie eine Materialstärke von 0,3 mm hat, können die Ringe die gleiche Materialstärke haben. Es ist aber auch möglich, für die Ringe eine Materialstärke von wesentlich mehr, beispielsweise 0,8, mm zu wählen. Der mögliche Bereich der Materialstärken von Ringen und Folie beträgt zwischen 0,05 mm und 1,2 mm, wobei in Einzelfällen nicht ausgeschlossen ist, das auch Materialstärken oberhalb oder unterhalb dieses Bereichs gewählt werden können.

Die Ringe haben in aufgeblasenem Zustand einen Querschnittsdurchmesser, der gegenüber dem nicht aufgeblasenem Zustand jedenfalls deutlich vergrößert ist. Typischerweise ist der Querschnittsdurchmesser kreisrund mit einer leichten Spitze zur Folie hin. Es ist aber auch möglich, durch entsprechende Wahl der Spritzgiessform den Querschnittsdurchmesser kreisförmig oder oval werden zu lassen, wenn die Ringe aufgeblasen sind.

Für die Herstellung der beiden Ringe ist es auch möglich, kurzerhand eine schlauchförmige Folie mit einem Durchmesser von beispielsweise 5 bis 10 cm endseitig je umzuschlagen, über beispielsweise 8 mm.

Das umgeschlagene Ende wird dann beispielsweise in einem Endbereich von 1 mm auf die Folie aufgeschweisst. Alternativ ist aus eine Verklebung mit einem Klebstoff möglich.

Beliebige weitere Modifikationen der Erfindung sind möglich, ohne den Bereich der Erfindung zu verlassen. Beispielsweise ist es möglich, in den Druckluftkanal zwischen Lippenring und Vestibulärring einen Druckminderer einzubauen. Dieser bewirkt dann, dass der Vestibulärring unter einem geringeren Überdruck steht als der Lippenring.

Weitere Vorteile, Einzelheiten und Merkmale ergeben sich aus der nachfolgenden Beschreibung mehrerer Ausführungsbeispiele der Erfindung anhand der Zeichnung.

Es zeigen:
- Fig.1: eine perspektivische Ansicht einer Ausführungsform eines erfindungsgemäßen Folienspannelements;
- Fig. 2: das Folienspannelement gemäß Fig. 1 jedoch in Rückansicht;
- Fig. 3: eine Vorderansicht des erfindungsgemäßen Folienspannelements in der Ausführungsform gemäß Fig. 1 und 2;
- Fig. 4: eine Seitenansicht des erfindungsgemäßen Folienspannelements in der Ausführungsform gemäß Fig. 1 und 2;
- Fig. 5: eine modifizierte Ausgestaltung des erfindungsgemäßen Folienspannelements;
- Fig. 6 bis Fig. 8: weitere Ansichten einer weiteren Ausführungsform eines erfindungsgemäßen Folienspannelements;
- Fig. 9: eine Schnittansicht durch das erfindungsgemäße Folienspannelement, entlang der Linie IX-IX, jedoch in einer modifizierten Ausführungsform; und
- Fig. 10: eine weitere Schnittansicht durch eine weitere Ausführungsform eines erfindungsgemäßen Folienspannelements.

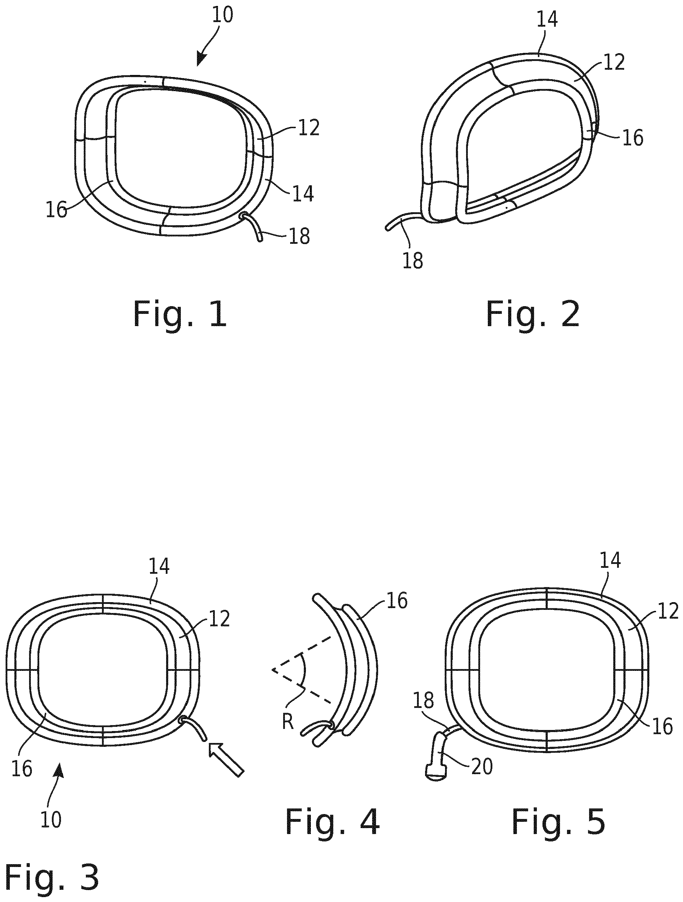

Aus Fig. 1 ist ein Folienspannelement 10 in dreidimensionaler Darstellung ersichtlich. Das Folienspannelement 10 ist in der Draufsicht im wesentlichen kreisförmig. Bei dem dargestellten Ausführungsbeispiel ähnelt die Form einem Viereck mit abgerundeten Ecken.

Anstelledessen ist aber auch eine beliebige andere Form möglich, beispielsweise eine kreisrunde Form oder eine ovale oder elliptische Form.

Günstig ist es, wenn eine gewisse Annäherung an die Form der Mundöffnung eines Patienten besteht.

Das Folienspannelement 10 weist eine hochelastischen Folie 12 auf, die sich zwischen einem Lippenring 14 und einem Vestibulärring 16 erstreckt. Beide Ringe 14 und 16 sind in Form von Schläuchen ausgebildet, die sich einstückig aus der Folie 12 heraus erstrecken. Der Lippenring 14 und der Vestibulärring 16 sind mit einem unter Druck stehenden Gas gefüllt. Bei dem Gas kann es sich um ein inertes Gas oder beispielsweise um Umgebungsluft handeln.

Bevorzugt sind die Ringe 14 und 16 aufblasbar. Hierzu weist das Folienspannelement 10 mindestens einen Druckluftanschluss 18 auf, wobei der Druckluftanschluss 18 aus Fig. 1 ersichtlich ist.

Der Druckluftanschluss 18 weist bevorzugt ein Rückschlagventil auf, das verhindert, dass ein aufgepumpter Ring 14 seine Luft verliert, wenn die Druckluftquelle, die für das Aufpumpen verwendet wird, entfernt wird.

In Fig. 1 und den weiteren Figuren ist lediglich ein Druckluftanschluss 18 dargestellt. Es versteht sich, dass für das Aufblasen des Vestibulärrings 16 ebenfalls Sorge getragen werden muss. Hierzu weist auch der Vestibulärring 16 einen Druckluftanschluss 18 auf, der sich beispielsweise von dem Vestibulärring 16 ausgehend oral entlang der Folie 12 erstrecken kann, bevorzugt seitlich.

Das Folienspannelement 10 ist in Fig. 1 von seiner Vorderseite dargestellt, und in Fig. 2 perspektivisch von seiner Rückseite. Der Lippenring 14 ist in an sich bekannter Weise etwas größer als der Vestibulärring 16. Bevorzugt springt die Folie 12 gegenüber dem Durchmesser der beiden Folien nach radial einwärts etwas zurück, so dass sie einen lippenkonformen Aufbau hat.

Für das Einsetzen des Folienspannelements 10 in den Mund des Patienten werden bevorzugt der Lippenring 14 und der Vestibulärring 16 etwas aufgeblasen, beispielsweise auf einen Überdruck von 100 mbar.

Das Folienspannelement 10 bleibt formstabil und lässt sich in den Mund des Patienten einführen. Nach dem Einführen wird das Folienspannelement 10 über den Druckluftanschluss 18 und gegebenenfalls einen weiteren Druckluftanschluss auf den erwünschten Überdruck aufgeblasen, beispielsweise auf 300 mbar.

In diesem Zustand erfolgt ein Aufspreizen und der Zahnarzt hat einen guten Zugang in den Mund des Patienten, auch beispielsweise, um die Vestibulärseite der Molaren über einen Intraoralscanner zu scannen.

Das Folienspannelement 10 ist bevorzugt in gleicher Materialstärke wie die Ringe 14 und 16hergestellt, und die beiden Ringe 14 und 16 sind einstückig zur Folie 12. Die Materialstärke kann in weiten Bereichen an die Erfordernisse angepasst werden und liegt im dargestellten Ausführungsbeispiel bei 0,2 mm.

Durch den erwünschten Überdruck dehnt sich jeder Ring 14 und 16 gegenüber dem biegeschlaffen und unaufgeblasenen Zustand aus, etwa auf das Doppelte seines Querschnittsdurchmessers im biegeschlaffen und unaufgeblasenen Zustand.

Der Lippenring 14 und der Vestibulärring 16 sind hierdurch, also durch den Luftdruck, ausgesteift, so dass sie die erwünschte Ersatzfunktion für harte Kunststoffringe übernehmen können, wie sie beim Stand der Technik vorgesehen sind und erfindungsgemäß vermieden werden können.

Aus Fig. 3 ist ein Folienspannelement 10 in der Ausführungsform gemäß den Fig. 1 und 2 in der Draufsicht ersichtlich. Es ist dargestellt, dass das Folienspannelement 10 in sich symmetrisch ist und etwas breiter als hoch, so dass es der Form der Mundöffnung etwas angepasst ist.

In Fig. 4 ist das Folienspannelement 10 in aufgeblasen Zustand der Ringe 14 und 16 ersichtlich. Wie es dargestellt ist, ist das gesamte Folienspannelement 10 um einen Radius R gekrümmt, der mehrere Zentimeter außerhalb der Mundöffnung und vor diesem liegt.

Die Folie 12 erstreckt sich etwas radial nach innen gekrümmt, so dass sie der Lippenform angepasst ist.

Aus Fig. 5 ist eine modifizierte Ausgestaltung eines erfindungsgemäßen Folienspannelements 10 ersichtlich. Der Druckluftanschluss 18 ist mit einem lösbarem Druckluftadapter 20 verbunden. Dieser dient dazu, die am Behandlungsplatz des Zahnarztpatienten vorhandene Druckluft zu verwenden, die Ringe 14 und 16 aufzublasen. Ferner ist bei dieser Lösung ein nicht dargestellter Druckluftkanal vorgesehen, der sich zwischen den Ringen 14 und 16 erstreckt.

Ferner ist bei dieser Ausführungsform der Querschnittsdurchmessers des Vestibulärrings 16 deutlich größer als der Querschnittsdurchmessers des Lippenrings 14. Der Vestibulärring 16 ist hierdurch etwas weicher und der Lippenring etwas härter.

Diese Verteilung der Steifheiten kann auch dadurch erreicht werden, dass der Lippenring 14 auf einen größeren Überdruck aufgeblasen wird als der Vestibulärring 16. Beispielsweise kann in dem Druckluftkanal zwischen den Ringen 14 und 16 ein Druckminderer vorgesehen sein, der dies leistet.

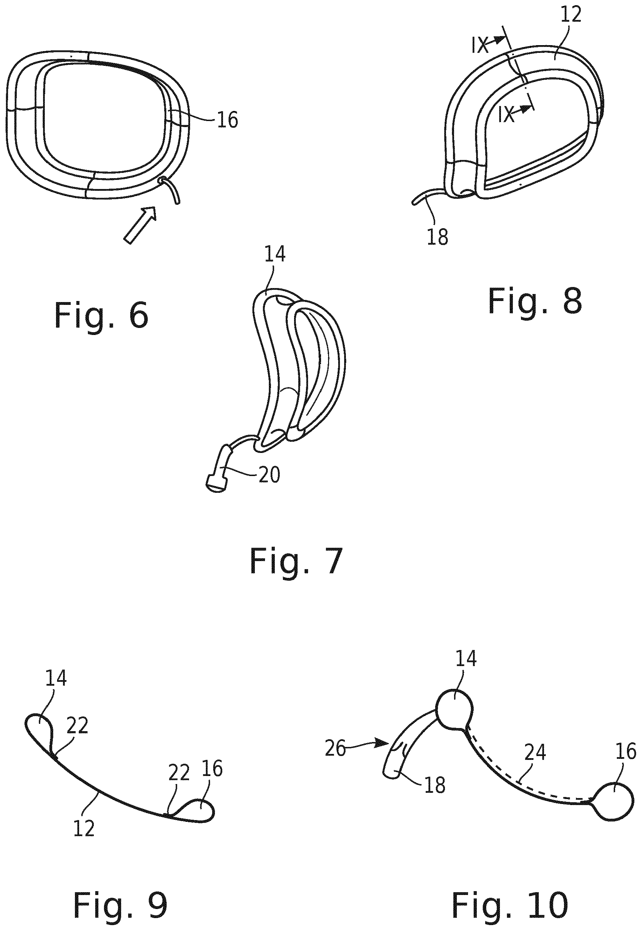

Aus den Fig. 6, 7 und 8 ist eine weitere Ausführungsform des erfindungsgemäßen Folienspannelements 10 ersichtlich. Durch die Wölbung des Folienspannelements 10 in aufgeblasenem Zustand lässt sich das Folienspannelement 10 besonders leicht in den Mund des Patienten einführen.

Der Zahnarzt drückt mittels seines Daumens und Zeigefingers etwa in der Sagittalebene die Ringe 14 und 16 etwas zusammen, so dass das Folienspannelement 10 noch breiter wird, aber weniger hoch. Dann führt er erst eine Seite des distalen Endes des Vestibulärrings 16 in das Vestibulum des Patienten ein, dann den mesialen Bereich, also den Bereich vor den Schneidezähnen, und nach Reduktion des Drucks zwischen Daumen und Zeigefingers dem anderen distalen Bereich, der durch das Vermindern des Drucks sich etwas nach mesial bewegt, so dass das Einführen erleichtert wird.

Wenn der Zahnarzt den Druck auf den Lippenring 14 oben und unten vollständig aufhebt, passt sich das erfindungsgemäße Folienspannelement 10 automatisch an die Mundform des Patienten an, denn der ausgeübte Druck wirkt vergleichmäßigend und wird vom Patienten deswegen als nicht störend empfunden, im Gegensatz zu harten Kunststoffringen, die Druckstellen und sogar Verletzungen erzeugen könnten.

Ein Schnitt durch das erfindungsgemäße Folienspannelement 10 entlang der Linie IX-IX ist aus Fig. 9 ersichtlich. Der Schnittverlauf ist in Fig. 8 angedeutet.

Bei dieser Ausführungsform ist es vorgesehen, dass die Folie 12 endseitig bei der Herstellung umgeschlagen wird, und hierdurch den Lippenring 14 und den Vestibulärring 16 bildet. Das umgeschlagene Ende bzw. dessen Endbereich 22 wird über eine kurze Strecke mit der Folie 12 verschweißt, indem das Ende dort auf die Folie 12 aufgedrückt wird, während sie erkaltet.

Bei dieser Ausführungsform erhalten der Lippenring 14 und der Vestibulärring 16 eine etwas tropfenförmige Form, wie es aus Fig. 9 ersichtlich ist.

Es versteht sich, das entsprechend den Fig. 1 bis 8 an mindestens einem der Ringe 14 und 16 ein Druckluftanschluss 18 angebracht ist, und das jeder Ring 14 und 16 einen geschlossen Hohlraum bildet, wenn er unter Druck steht.

In Fig. 10 ist eine weitere Ausführungsform dargestellt. Dort ist ersichtlich, in welcher Weise sich ein Druckluftkanal 24 zwischen den Ringen 14 und 16 entlang der Folie 12 erstrecken kann. Der Druckluftkanal 24 kann auch in die Folie 12 eingearbeitet sein und sich ebenfalls einstückig mit dieser und den Ringen 14 und 16 erstrecken.

Der Druckluftanschluss 18 kann das aus Fig. 10 schematisch ersichtliche Rückschlagventil 26 aufweisen, das verhindert, dass die eingebrachte Druckluft ausströmt, wenn die Druckluftquelle entfernt wird.

Soll einmal doch etwas Druckluft abgelassen werden, kann der Zahnart die Stelle des Rückschlagventils 26 in den Druckluftkanal 24 etwas zwischen Daumen und Zeigefinger zusammendrücken, so dass das Rückschlagventil 26 nicht mehr in Funktion ist und etwas Luft abgelassen werden kann.

Das erfindungsgemäße Folienspannelement 10 ist besonders leicht und preisgünstig herzustellen, nachdem es ausschließlich aus der hochelastischen Folie 12 besteht, die auch mit konstanter Folienstärke eingesetzt werden kann.

## Patentansprüche

1. Folienspannelement, mit einem Lippenring und einem Vestibulärring, zwischen denen, und gegebenenfalls über welchen hinaus, sich eine Folie erstreckt, **dadurch gekennzeichnet, dass** mindestens der Vestibulärring (16) und/oder der Lippenring (14) mit einem unter Druck stehenden Gas gefüllt ist.

2. Folienspannelement nach einem der vorherigen Ansprüche, **dadurch gekennzeichnet, dass** sowohl der Lippenring (14) als auch der Vestibulärring (16) mit Gas gefüllt sind.

3. Folienspannelement nach einem der vorherigen Ansprüche, **dadurch gekennzeichnet, dass** das Gas Luft, insbesondere Umgebungsluft, ist.

4. Folienspannelement nach einem der vorherigen Ansprüche, **dadurch gekennzeichnet, dass** der Lippenring (14) und/oder der Vestibulärring (16) aufblasbar sind.

5. Folienspannelement nach einem der vorherigen Ansprüche, **dadurch gekennzeichnet, dass** der Lippenring (14) und/oder der Vestibulärring (16) je aus einem Schlauch bestehen oder besteht, welcher Schlauch mit der Folie (12) verbunden ist, die sich zwischen den Ringen (14,16) erstreckt.

6. Folienspannelement nach einem der vorherigen Ansprüche, **dadurch gekennzeichnet, dass** die Ringe (14,16) und die Folie (12) miteinander einstückig sind und insbesondere aus dem gleichen Material bestehen.

7. Folienspannelement nach einem der vorherigen Ansprüche, **dadurch gekennzeichnet, dass** der Lippenring (14) einen Druckluftanschluss (18) aufweist, über welchen er aufblasbar ist.

8. Folienspannelement nach einem der vorherigen Ansprüche, **dadurch gekennzeichnet, dass** das Gas, insbesondere die Luft, in beiden Ringen (14,16) miteinander in Strömungsverbindung steht, insbesondere über einen Druckluftkanal (24).

9. Folienspannelement nach einem der vorherigen Ansprüche, **dadurch gekennzeichnet, dass** ein Druckluftanschluss (18) mit einer insbesondere einstellbaren Druckluftquelle in Verbindung bringbar ist oder steht, über welche der Vestibulärring (16) und/oder der Lippenring (14) unter einen individuell einstellbaren Druck bringbar ist.

10. Folienspannelement nach einem der vorherigen Ansprüche, **dadurch gekennzeichnet, dass** sowohl der Lippenring (14) als auch der Vestibulärring (16) als auch die Folie (12) aus einem hoch elastischem Material besteht und beim Aufblasen der Ringe (14,16) sich sowohl deren Durchmesser als auch der Durchmesser des Folienspannelements (10) vergrößert.

11. Folienspannelement nach einem der vorherigen Ansprüche, **dadurch gekennzeichnet, dass** ein Druckluftanschluss (18) vorgesehen ist, über welchen bei Bedarf auch Druckluft aus dem Lippenring (14) und/oder Vestibulärring (16) ablassbar ist.

12. Folienspannelement nach einem der vorherigen Ansprüche, **dadurch gekennzeichnet, dass** die Ringe (14,16) mit einem Überdruck von 50 mbar bis 200 mbar aufblasbar sind und beim Aufblasen ihr Volumen - betrachtet gegenüber dem entspannten Zustand - auf ein Vielfaches vergrößern, insbesondere mindestens um das Dreifache.

13. Folienspannelement nach einem der vorherigen Ansprüche, **dadurch gekennzeichnet, dass** der Vestibulärring (16) und/oder der Lippenring (14) in aufgeblasenem Zustand einen Durchmesser hat bzw. einen Durchmesser haben, der mindestens einem Zehntel des Abstands zwischen den Ringen (14,16) bei gestreckter Folie (12) entspricht, und höchstens einem Drittel dieses Abstands.

14. Folienspannelement nach einem der vorherigen Ansprüche, **dadurch gekennzeichnet, dass** der Druckluftanschluss (18) im Bereich der Unterlippe am Lippenring (14) angebracht ist, insbesondere seitlich der Frontzähne des Patienten.

15. Folienspannelement nach einem der vorherigen Ansprüche, **dadurch gekennzeichnet, dass** die Materialstärke der Ringe (14,16) unterschiedlich ist, insbesondere die des Vestibulärringes (16) größer als der des Lippenringes (14).

16. Folienspannelement nach einem der vorherigen Ansprüche, **dadurch gekennzeichnet, dass** die Ringe (14,16) bei der Herstellung durch ein Umschlagen und Befestigen, insbesondere Aufschweißen, der Folie (12) gebildet sind.

17. Folienspannelement nach einem der vorherigen Ansprüche, **dadurch gekennzeichnet, dass** der Vestibulärring (16) unter einem geringeren Überdruck als der Lippenring (14) steht, und einen eigenen Druckluftanschluss (18) oder einen Tochter-Druckluftanschluss, insbesondere einen Druckminderer, aufweist.

18. Folienspannelement nach einem der vorherigen Ansprüche, **dadurch gekennzeichnet, dass** ein Druckluftadapter (20) vorgesehen ist mit welchem ein Druckluftanschluss (18) des Folienspannelements (10) an den Druckluftauslass an einem zahnärztlichen Behandlungsplatz anschließbar ist.

## Claims

1. A film tensioning element, having a lip ring and a vestibular ring, between which, and optionally beyond which, a film extends, **characterized in that** at least the vestibular ring (16) and/or the lip ring (14) is filled with a gas under pressure.

2. The film tensioning element according to one of the preceding claims, **characterized in that** both the lip ring (14) and the vestibular ring (16) are filled with gas.

3. The film tensioning element according to one of the preceding claims, **characterized in that** the gas is air, in particular ambient air.

4. The film tensioning element according to one of the preceding claims, **characterized in that** the lip ring (14) and/or the vestibular ring (16) are inflatable.

5. The film tensioning element according to one of the preceding claims, **characterized in that** the lip ring (14) and/or the vestibular ring (16) consist(s) of a tube, respectively, said tube being connected to the film (12) extending between the rings (14,16).

6. The film tensioning element according to one of the preceding claims, **characterized in that** the rings (14,16) and the film (12) are integral with each other and in particular consist of the same material.

7. The film tensioning element according to one of the preceding claims, **characterized in that** the lip ring (14) has a compressed air connection (18) via which it can be inflated.

8. The film tensioning element according to one of the preceding claims, **characterized in that** the gas, in particular the air, in both rings (14,16) is in flow connection with one another, in particular via a compressed air channel (24).

9. The film tensioning element according to one of the preceding claims, **characterized in that** a compressed air connection (18) is or can be connected to an in particular adjustable source of compressed air, via which the vestibular ring (16) and/or the lip ring (14) can be brought under an individually adjustable pressure.

10. The film tensioning element according to one of the preceding claims, **characterized in that** both the lip ring (14) and the vestibular ring (16) as well as the film (12) consist of a highly elastic material and when the rings (14,16) are inflated both their diameter and the diameter of the film tensioning element (10) increase.

11. The film tensioning element according to one of the preceding claims, **characterized in that** a compressed air connection (18) is provided, via which compressed air can also be released from the lip ring (14) and/or vestibular ring (16) if required.

12. The film tensioning element according to one of the preceding claims, **characterized in that** the rings (14,16) can be inflated with an overpressure of 50 mbar to 200 mbar and when inflated increase their volume - considered in comparison with the relaxed state - to a multiple, in particular at least by three times.

13. The film tensioning element according to one of the preceding claims, **characterized in that** the vestibular ring (16) and/or the lip ring (14) in the inflated state has or have a diameter which corresponds to at least one tenth of the distance between the rings (14,16) when the film (12) is stretched, and one third of this distance at most.

14. The film tensioning element according to one of the preceding claims, **characterized in that** the compressed air connection (18) is attached to the lip ring (14) in the region of the lower lip, in particular laterally to the patient's front teeth.

15. The film tensioning element according to one of the preceding claims, **characterized in that** the material thickness of the rings (14,16) is different, in particular that of the vestibular ring (16) is greater than that of the lip ring (14) .

16. The film tensioning element according to one of the preceding claims, **characterized in that** the rings (14,16) are formed during manufacture by folding over and fastening, in particular welding on, the film (12).

17. The film tensioning element according to one of the preceding claims, **characterized in that** the vestibular ring (16) is under a lower overpressure than the lip ring (14) and has its own compressed air connection (18) or a subsidiary compressed air connection, in particular a pressure reducer.

18. The film tensioning element according to one of the preceding claims, **characterized in that** a compressed air adapter (20) is provided with which a compressed air connection (18) of the film tensioning element (10) can be connected to the compressed air outlet at a dental treatment station.

## Revendications

1. Élément de tension de feuille, avec un anneau labial et un anneau vestibulaire, entre lesquels, et éventuellement au-delà desquels, s'étend une feuille, **caractérisé en ce qu'**au moins l'anneau vestibulaire (16) et/ou l'anneau labial (14) est rempli d'un gaz sous pression.

2. Élément de tension de feuille selon l'une quelconque des revendications précédentes, **caractérisé en ce que** l'anneau labial (14) et l'anneau vestibulaire (16) sont tous deux remplis de gaz.

3. Élément de tension de feuille selon l'une des revendications précédentes, **caractérisé en ce que** le gaz est de l'air, en particulier de l'air ambiant.

4. Élément de tension de feuille selon l'une des revendications précédentes, **caractérisé en ce que** l'anneau labial (14) et/ou l'anneau vestibulaire (16) sont gonflables.

5. Élément de tension de feuille selon l'une des revendications précédentes, **caractérisé en ce que** l'anneau labial (14) et/ou l'anneau vestibulaire (16) sont ou sont chacun constitués d'un tube, lequel tube est relié à la feuille (12) qui s'étend entre les anneaux (14, 16).

6. Élément de tension de feuille selon l'une des revendications précédentes, **caractérisé en ce que** les anneaux (14, 16) et la feuille (12) sont d'un seul tenant l'un avec l'autre et sont en particulier constitués du même matériau.

7. Élément de tension de feuille selon l'une des revendications précédentes, **caractérisé en ce que** l'anneau labial (14) présente un raccord d'air comprimé (18) par lequel il peut être gonflé.

8. Élément de tension de feuille selon l'une des revendications précédentes, **caractérisé en ce que** le gaz, en particulier l'air, est en communication fluidique entre les deux anneaux (14, 16), en particulier par l'intermédiaire d'un canal d'air comprimé (24).

9. Élément de tension de feuille selon l'une des revendications précédentes, **caractérisé en ce qu'**un raccord d'air comprimé (18) peut être ou est mis en liaison avec une source d'air comprimé, en particulier réglable, par laquelle l'anneau vestibulaire (16) et/ou l'anneau labial (14) peut être mis sous une pression réglable individuellement.

10. Élément de tension de feuille selon l'une des revendications précédentes, **caractérisé en ce que** tant l'anneau labial (14) que l'anneau vestibulaire (16) ainsi que la feuille (12) sont constitués d'un matériau hautement élastique et que, lors du gonflage des anneaux (14, 16), tant leur diamètre que le diamètre de l'élément de tension de feuille (10) s'agrandissent.

11. Élément de tension de feuille selon l'une des revendications précédentes, **caractérisé en ce qu'**un raccord d'air comprimé (18) est prévu, par lequel de l'air comprimé peut également être évacué de l'anneau labial (14) et/ou de l'anneau vestibulaire (16) en cas de besoin.

12. Élément de tension de feuille selon l'une des revendications précédentes, **caractérisé en ce que** les anneaux (14, 16) peuvent être gonflés avec une surpression de 50 mbar à 200 mbar et, lors du gonflage, augmentent leur volume - considéré par rapport à l'état détendu - d'un multiple, en particulier d'au moins trois fois.

13. Élément de tension de feuille selon l'une des revendications précédentes, **caractérisé en ce que** l'anneau vestibulaire (16) et/ou l'anneau labial (14) a ou ont, à l'état gonflé, un diamètre correspondant à au moins un dixième de la distance entre les anneaux (14, 16) lorsque la feuille (12) est étirée, et au plus un tiers de cette distance.

14. Élément de tension de feuille selon l'une des revendications précédentes, **caractérisé en ce que** le raccord d'air comprimé (18) est placé dans la zone de la lèvre inférieure sur l'anneau labial (14), en particulier sur le côté des dents antérieures du patient.

15. Élément de tension de feuille selon l'une des revendications précédentes, **caractérisé en ce que** l'épaisseur de matériau des anneaux (14, 16) est différente, en particulier celle de l'anneau vestibulaire (16) est supérieure à celle de l'anneau labial (14).

16. Élément de tension de feuille selon l'une des revendications précédentes, **caractérisé en ce que** les anneaux (14, 16) sont formés lors de la fabrication par un rabattement et une fixation, en particulier par soudage, de la feuille (12) .

17. Élément de tension de feuille selon l'une des revendications précédentes, **caractérisé en ce que** l'anneau vestibulaire (16) est soumis à une surpression inférieure à celle de l'anneau labial (14), et comporte un raccord d'air comprimé (18) propre ou un raccord d'air comprimé fils, en particulier un détendeur.

18. Élément de tension de feuille selon l'une des revendications précédentes, **caractérisé en ce qu'**un adaptateur d'air comprimé (20) est prévu, avec lequel un raccord d'air comprimé (18) de l'élément de tension de feuille (10) peut être raccordé à la sortie d'air comprimé d'un poste de traitement dentaire.
